## Europäisches Patentamt
## European Patent Office
## Office européen des brevets

(19)

(11) Veröffentlichungsnummer : **0 304 452 B1**

(12)

# EUROPÄISCHE PATENTSCHRIFT

(45) Veröffentlichungstag der Patentschrift :
22.01.92 Patentblatt 92/04

(51) Int. Cl.⁵ : **A61F 5/455**

(21) Anmeldenummer : 88902093.9

(22) Anmeldetag : 05.03.88

(86) Internationale Anmeldenummer :
**PCT/DE88/00119**

(87) Internationale Veröffentlichungsnummer :
**WO 88/06874 22.09.88 Gazette 88/21**

(54) URINABLEITUNGSVORRICHTUNG FÜR HARNINKONTINENTE WEIBLICHE PERSONEN.

(30) Priorität : 13.03.87 DE 3708181
22.03.87 DE 3709439

(43) Veröffentlichungstag der Anmeldung :
01.03.89 Patentblatt 89/09

(45) Bekanntmachung des Hinweises auf die
Patenterteilung :
22.01.92 Patentblatt 92/04

(84) Benannte Vertragsstaaten :
AT BE CH DE FR GB IT LI LU NL SE

(56) Entgegenhaltungen :
GB-A- 1 422 638
GB-A- 2 072 512
US-A- 4 610 675

(73) Patentinhaber : SCHAUF, Wilhelm
Ostpreussenweg 3
W-2841 Wagenfeld 1 (DE)

(72) Erfinder : SCHAUF, Wilhelm
Ostpreussenweg 3
W-2841 Wagenfeld 1 (DE)

(74) Vertreter : Thömen, Uwe, Dipl.-Ing.
Patentanwalt U. Thömen Zeppelinstrasse 5
W-3000 Hannover 1 (DE)

EP 0 304 452 B1

**Beschreibung**

Die Erfindung betrifft eine Urinableitungsvorrichtung für harninkontinente weibliche Personen nach dem Oberbegriff des Anspruchs 1; wie sie durch die GB-1 422 638 bekannt ist.

Eine derartige Vorrichtung dient dazu, den betreffenden Personen unkontrolliert abgehenden Urin in einem Urinbeutel aufzufangen, ohne daß dabei Urin danebenläuft und zu Durchfeuchtungen der Kleidung und Geruchsbelästigung führt.

Im Gegensatz zu Harninkontinenten männlichen Personen ist eine sichere Abdichtung und eine beim Tragen möglichst wenig störende Ausgestaltung aus anatomischen Gründen wesentlich schwieriger zu erreichen.

Aus der WO 85/05264 ist eine als Urin-Sammler bezeichnete Vorrichtung bekannt, die einen durch Leitgürtel am Körper befestigten Zuschnitt umfaßt, auf dem sogenannte Kollektor-Strukturen angeordnet sind. Mittels der Leitgürtel wird der Zuschnitt so am Körper der Trägerin befestigt, daß die Kollektor-Struktur zwischen den äußeren Schamlippen zu liegen kommt. Die Kollektor-Struktur ist dabei als zick-zack-förmige Erhebung ausgebildet, die eine ovale Form aufweist und sich längs der Schamlippen der Trägerin erstreckt. Auf der Unterseite befindet sich ein ovales, sich nach unten verjüngendes Anschlußstück.

Der gesamte von den Erhebungen der Kollektor-Struktur umschlossene Bereich bildet eine Feuchtzone, die von Urin benetzt wird. Außerdem ist auf dem Zuschnitt ein umlaufender Wulst angeordnet, der bei Anlage des Urinsammlers außerhalb der äußeren Schamlippen Hautkontakt bekommt und eine Abdichtung nach außen bewirkt.

Durch die Befestigung des Urinsammlers mittels Leitgürteln werden verhältnismäßig weit von der Harnaustrittsöffnung der Trägerin liegende Körperteile zur Festlegung des Urinsammlers benutzt. Eine Verlagerung dieser Körperteile führt deshalb auch zu einer entsprechenden Verlagerung des Urinsammlers, so daß eine gleichbleibende Lage bei Bewegungen nicht gewährleistet ist. Zwar stellt die Ausgestaltung der Kollektor-Strukturen eine zusätzliche Positionierhilfe dar, die verhältnismäßig schmale Ausgestaltung der Erhebungen übt aber besonders bei Verlagerungen einen Druck auf die Schamlippen aus, der als unangenehm empfunden wird. Außerdem ist durch die ovale Formgebung der Kollektor-Struktur auch ein großer Teil der Vagina als Feuchtbereich ständig mit Urin in Kontakt, so daß dort Hautreizungen entstehen können.

Ferner ist aus der DE-OS 20 43 198 ein Urinauffangbehälter bekannt, der mittels Träger am Körper der Trägerin befestigt wird. Zur Abdichtung gegen den Körper dient ein Schaumstoffkissen, das eine längliche, dem Vaginalbereich zugewandte Öffnung aufweist.

Mittel zum Spreizen der äußeren Schamlippen sind hier nicht vorgesehen. Der Urin kann daher nicht gezielt von der Harnröhre in den Auffangbehälter übertreten, sondern läuft unkontrolliert zwischen den geschlossenen Schamlippen ab. Diese sind also ständig von Urin befeuchtet, was auch bei dieser bekannten Vorrichtung zu Hautreizungen führen kann. Durch die Träger wird außerdem auch die Lage des Auffangbehälters von der Lage weit entfernter Körperteile abhängig, so daß dieser bei Bewegungen hin- und herrutschen kann. Die Dichtwirkung des Schaumstoffes ist materialbedingt unzureichend, besonders wenn der Auffangbehälter mehrfach verrutscht.

Durch das Dokument GB-14 22 638, von dem die Erfindung ausgeht, ist auch schon eine Urinableitungsvorrichtung für harninkontinente weibliche Personen bekannt, wobei die Vorrichtung eine zur Spreizung der äußeren Schamlippen bestimmte Erhebung aufweist, die als zentral zwischen die äußeren Schamlippen zu liegen kommende Nase ausgebildet ist, welche die Abflußöffnung zum Vaginalbereich hin begrenzt.

Die als Nase ausgebildete Erhebung spreizt die Schamlippen, so daß ein freier Austritt des Urins aus der Harnröhre möglich ist. Ferner begrenzt die Nase die Abflußöffnung und damit auch den Austrittsbereich für den Urin zum Vaginalbereich hin, so daß die Gefahr von Durchnässungen der über die Urinableitungsvorrichtung gezogenen Textilien verhindert werden kann. Als Folge davon lassen sich auch die durch durchsickernden Urin hervorgerufenen Geruchsbelästigungen verringern.

Diesen an sich erwünschten Eigenschaften steht bei der bekannten Urinableitungsvorrichtung allerdings der Nachteil entgegen, daß sie sich nicht zufriedenstellend befestigen läßt. Ferner bereitet es Probleme, das Formstück individuell an die Gegebenheiten verschiedener weiblicher Personen anzupassen. Bei einer mangelhaften Befestigung besteht aber wieder die Gefahr von Geruchsbelästigungen infolge von durchsickerndem Urin.

Der Erfindung liegt die Aufgabe zugrunde, ein Formstück zu schaffen, welches eine sicher haltbare Befestigung mit der Möglichkeit einer individuellen Anpassung des Formstückes an die Bedürfnisse der jeweiligen Trägerin ermöglicht.

Diese Aufgabe wird bei der im Oberbegriff des Anspruchs 1 genannten Vorrichtung durch die im kennzeichnenden Teil angegebenen Merkmale gelöst.

Die neue Urinableitungsvorrichtung läßt sich sicher und individuell am Körper der Trägerin befestigen. Die Klebeschicht zur Befestigung des Formstückes ermöglicht es, das Formstück vor dem Aufkleben individuell in

2

der Weise örtlich zu fixieren, daß die Abflußöffnung mit dem Harnröhrenausgang fluchtet.

Von besonderem Vorteil ist es, wenn gemäß einer zweckmäßigen Ausgestaltung der Erfindung die Klebeschicht durch ein doppelseitiges Klebeband gebildet wird.

Zunächst wird die Klebeschicht mit ihrer einen Seite im Schritt der Trägerin auf die Haut geklebt, und im Anschluß daran wird auf der anderen Seite der Klebeschicht das Formstück aufgeklebt, welches sich in der beschriebenen Weise vorteilhaft örtlich fixieren läßt. Hierbei ist im Bereich der durch die ovale Zone begrenzten Öffnung in jedem Fall eine individuelle Anpassung möglich, denn die Trägerin kann die Abflußöffnung innerhalb dieses Bereiches entsprechend den persönlichen Bedürfnissen variieren und der örtlichen Lage des Harnröhrenausgangs anpassen. Durch das doppelseitige Klebeband wird zudem eine sehr sichere Befestigung gewährleistet.

Die bei der neuen Urinableitungsvorrichtung vorgesehene Art der Befestigung bietet wesentliche Vorteile gegenüber der Festlegung mit Hilfe von Träger oder Gürtel. Während letztere nämlich bei Bewegungen der von der Harnröhre weit entfernten Körperteile eine starke Verlagerung der Vorrichtung herbeiführen, ist dies bei Befestigung unmittelbar an der Harnröhre mit Hilfe des doppelseitigen Klebebandes wesentlich weniger der Fall. Die Klebeschicht bildet ferner eine sichere Abdichtung gegen einen möglichen Rest von nicht durch die Abflußöffnung ablaufenden Urin.

Dabei unterstützen sich die Maßnahmen und ihre Wirkungen gegenseitig. Da nämlich keine Verlagerung bei Bewegungen auftritt, bleibt auch die Klebestelle ortsfest und wird nicht durch eindringenden Urin aufgeweicht. Dies wiederum führt zu einer sehr dauerhaften und allseitig guten Verbindung. Da die von der Trägerin besonders gefürchtete Geruchsbelästigung sicher vermieden wird, braucht sie sich auch in ihrer Bewegungsfreiheit nicht einzuschränken.

Gemäß einer Weiterbildung der Erfindung ist innerhalb des von der ovalen Zone umgrenzten Bereiches am Fuß des Nasenrückens eine Zusatzöffnung angeordnet, der sich ein weiterer Abflußschlauch anschließt.

Durch diese als zusätzliche Sicherheit gedachte Maßnahme soll Urin abgeleitet werden, der beispielsweise durch unsachgemäßes Anlegen der Vorrichtung nicht durch die Abflußöffnung abgeflossen ist, sondern zwischen Schamlippen und Nase entlang gewandert ist und sich im unteren Bereich der ovalen Zone sammelt und die Klebeschicht aufweichen könnte.

In Weiterbildung dieser Maßnahme vereinigen sich die Abflußschläuche auf dem Wege zum Urinbeutel. Zum einen wird durch diese Maßnahme ein unmittelbarer Rückfluß des durch die Abflußöffnung in das Anschlußstück laufenden Urins verhindert, und zum anderen bewirkt der durch den Hauptabflußschlauch laufende Urin in dem anderen Abflußschlauch einen gewissen Unterdruck, der beim Harnlassen den eventuell am Fuße des Nasenrückens angesammelten Urin absaugt.

Bei einer weiteren vorteilhaften Ausführungsform der Urinableitungsvorrichtung sind auf beiden Seiten des Formstückes in Höhe der Nase Verlängerungsansätze angeordnet. Diese sind zur Anlage an den Innenseiten der Oberschenkel der Trägerin bestimmt. Durch die Verlängerungsansätze wird eine gute Lagestabilisierung der Vorrichtung in Längsrichtung bewirkt, bei der die Nase als stabilisierendes Element nicht ausreicht und die Klebeschicht in ihrer stabilisierenden Wirkung wirksam unterstützt wird. Außerdem verhindern die Verlängerungsansätze in ihrem Obergangsbereich zu dem Formstück, daß die seitlichen Kanten des Formstückes scharfkantig in Hautfalten der Oberschenkel eindringen.

In Weiterbildung der zuvor beschriebenen Maßnahme besitzen die Verlängerungsansätze einen Zuschnitt, der sich von einem schmalen Steg zur Befestigung am Formstück in Richtung auf sein freies Ende verbreitert.

Hierdurch wird eine großflächige Anlage am Oberschenkel der Trägerin erreicht, ohne daß das Einknicken des Oberschenkels behindert wird oder zu einem Verrutschen des Formstückes führt. Vielmehr können sich die Verlängerungsansätze gegenüber dem Formstück auch etwas in Längsrichtung des Formstückes verschwenken.

Bei einer weiteren praktischen Ausgestaltung der Erfindung sind die mit der Haut der Trägerin unmittelbar kontakthaltenden Bereiche des Formstückes und der Verlängerungsansätze mit einer Transpiration aufnehmenden Textilie beschichtet.

Hierdurch wird einmal ein Schwitzen verhindert, und zum anderen läßt sich ein Verrutschen der Vorrichtung auf dem Körper begünstigender Transpirationsfilm beseitigen. Somit trägt auch diese Maßnahme besonders bei schwüler Witterung zu einer sicheren Funktion der neuen Urinableitungsvorrichtung bei.

Nachfolgend wird die Erfindung anhand des in der Zeichnung dargestellten Ausführungsbeispiels näher erläutert.

Es zeigen:

Fig. 1 eine perspektivische Ansicht der Urinableitungsvorrichtung in der Anlegestellung,

Fig. 2 einen Ausschnitt aus dem vorderen Teil der in Fig. 1 dargestellten Vorrichtung in Verbindung mit einer Anlegehilfe, und

Fig. 3 die in Fig. 1 dargestellte Vorrichtung am Körper einer Trägerin angelegt.

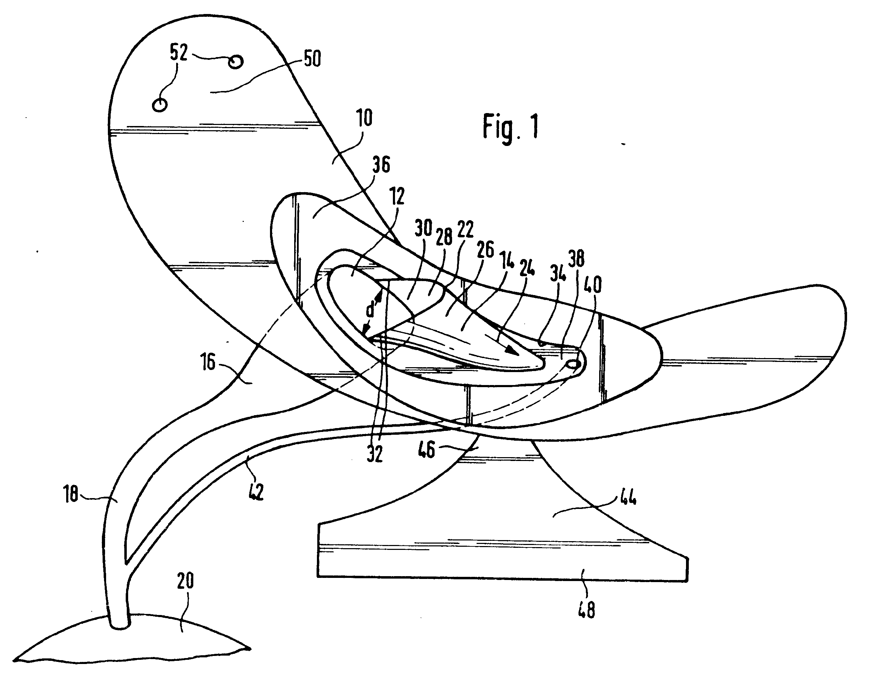

Die in Fig. 1 dargestellte Urinableitungsvorrichtung umfaßt als Basis ein Formstück 10 aus flexiblem Material, vorzugsweise aus Kunststoff. Das Formstück 10 weist einen länglichen, annähernd herzähnlichen Zuschnitt auf und ist konkav vorgewlbt, um sich an die Körperform einer Trägerin gut anpassen zu können. In das Formstück 10 ist eine Abflußöffnung 12 eingelassen, der sich ein kelchförmiges Anschlußstück 16 anschließt. Dieses geht in einen Abflußschlauch 18 über, der wiederum in einen Urinbeutel 20 mündet.

Der Abflußöffnung 12 schließt sich rücklings eine Nase 14 an, die mit ihrer Nasenfront 28 die Abflußöffnung 12 begrenzt, dort ihre höchste Erhebung 22 aufweist und deren Nasenrücken 26 im weiteren Verlauf 24 des Vaginalbereichs schräg bis auf das Höhenniveau des Formstücks 10 abfällt. Die Nasenfront 28 ist konkav geformt und bildet einen Einlauftrichter 30, wobei ihre seitlichen Flanken 32 die Abflußöffnung 12 etwa bis zur Hälfte ihres Umfangs umschließen. Die Abflußöffnung 12 weist den Durchmesser d auf, der etwa nach dem Austrittskegel des Urinstrahls aus der Harnröhre bemessen ist.

Die Abflußöffnung 12 sowie die Nase 14 sind von einer ovalen Zone 34 umgeben. Diese ovale Zone 34 trägt eine Klebeschicht, die als doppelseitiges Klebeband 36 ausgebildet ist. Im Innenraum des von der ovalen Zone 34 eingeschlossenen Bereichs, und zwar am Fuße 38 des Nasenrückens 26, befindet sich eine Zusatzöffnung 40, die über einen weiteren Abflußschlauch 42 mit dem Abflußschlauch 18 verbunden ist.

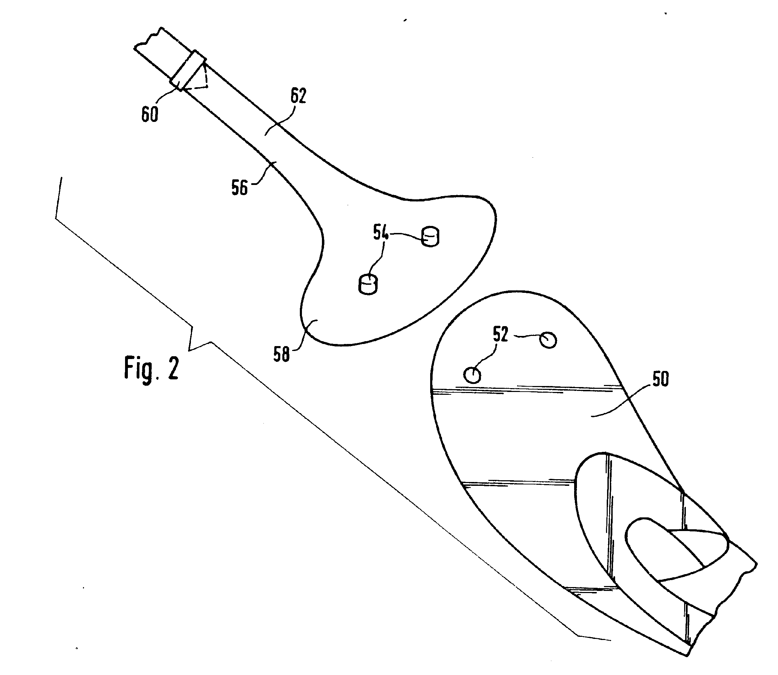

An dem Formstück 10 sind zu beiden Seiten Verlängerungsansätze 44 angeordnet. Diese umfassen an der Verbindungsstelle mit dem Formstück 10 einen schmalen Steg und erweitern sich in Richtung auf das freie Ende 48. Der vordere Teil des Formstückes 10 ist sehr großflächig ausgebildet und besitzt die Gestalt eines Latzes 50. In diesem Latz 50 sind Löcher 52 vorgesehen, die, die.Fig. 2 zeigt, die Zapfen 54 einer Anlegehilfe 56 aufnehmen können. Die Anlegehilfe 56 besteht aus einer Platte 58, auf der die Zapfen 54 angeordnet sind und umfaßt einen Meßstreifen 62 mit einer Markierung 60. Die Markierung 60 kann dabei verschiebbar auf dem Meßstreifen 62 angeordnet sein.

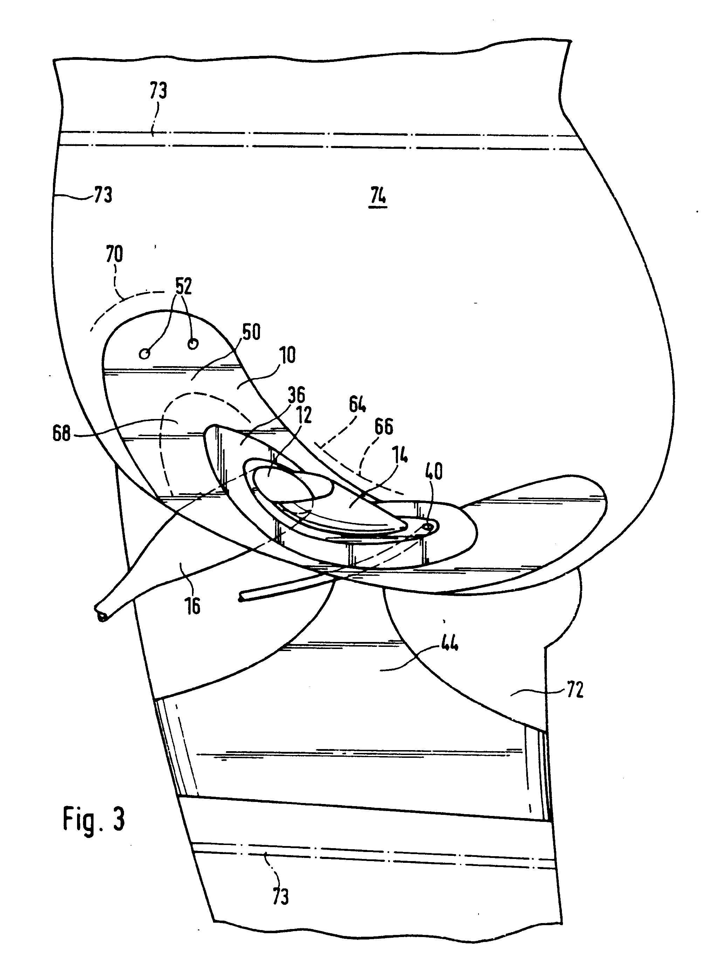

Fig. 3 zeigt die erfindungsgemäße Urinableitungsvorrichtung an einer Trägerin 74 angelegt. Der besseren Übersicht halber sind nicht alle in Fig. 1 gezeigten Bezugszeichen hier noch einmal wiederholt. Vielmehr beschränkt sich die Darstellung auf die wichtigsten Bezugszeichen der Fig. 1 und umfaßt noch zusätzliche Bezugszeichen zur Kennzeichnung der Körperbereiche der Trägering 74.

Die Vorrichtung wird so angelegt, daß die Abflußöffnung 12 mit der Harnröhrenöffnung 64 genau fluchtet. Die Nase 14 kommt dabei zwischen die großen Schamlippen im Vaginalbereich 66 zu liegen. Durch die konvexe Ausgestaltung des Nasenrückens 26 können sich die Schamlippen der Nasenform gut anpassen, ohne daß dabei Druckstellen zu befürchten sind. Die Vorrichtung wird auf diese Weise seitlich sehr genau fixiert.

Entsprechend der Größe der Schamlippen wird das doppelseitige Klebeband 36 zugeschnitten und angepaßt, so daß es genau außerhalb der Schamlippen mit der diese umgebenden Haut in Kontakt kommt. Der von der ovalen Zone 34 eingeschlossene Bereich ist dann urindicht gegen den Außenraum abgeschlossen. Das Klebeband 36 ist dabei so beschaffen, daß ein Verrutschen sicher verhindert wird, ein Abziehen der Urinableitungsvorrichtung nach eintägigem Tragen aber ohne Reizung der beklebten Hautpartien möglich ist. Das doppelseitige Klebeband 36 verhindert, daß Urin nach außen tritt und dort zu Geruchsbelästigung führt. Außerdem wird auch ein Unterwandern der Klebeschicht und eine damit einhergehende Schwächung der Klebewirkung verhindert, Die Lage des Klebebandes 36 auf dem Formstück 10 ist variabel wählbar. Liegt die Abflußöffnung 12 in Flucht mit der Harnröhrenöffnung, so ist durch die Spreizung der Schamlippen mittels der Nase 14 ein freier Austritt des Urins aus der Harnröhre möglich. Der Urinstrahl kann also unmittelbar in die Abflußöffnung 12 übertreten und über das kelchförmige Anschlußstück und den Abflußschlauch 16 in den Urinbeutel 20 laufen. Dabei verjüngt sich der Durchmesser d der Abflußöffnung 12 kontinuierlich auf den Durchmesser des Abflußschlauchs 18, ohne daß Hindernisse den freien Abfluß verhindern und einen Rückstau hervorrufen können.

Zur Leitung des Austrittskegels des Urinstrahls trägt auch die konkav geformte Nasenfront 28 bei, die zusammen mit den geöffneten Schamlippen einen Einlauftrichter 30 bildet. Der Einlauftrichter 30 geht dabei praktisch glattwandig durch die Abflußöffnung 12 in das kelchförmige Anschlußstück 16 über.

Der freie Urinabfluß verhindert, daß durch rückstauenden Urin die weiter im Vaginalbereich 66 gelegenen Schamlippen mit Urin benetzt werden und wund werden können. Für den Fall, daß durch eine ungünstige Lage beim Harn lassen dennoch Urin zwischen den Schamlippen und der Nase 14 hindurchsickert, wird diese Restmenge am Fuße 38 des Nasenrückens gesammelt und über die Zusatzöffnung 40 und den weiteren Abflußschlauch 42 ebenfalls dem Urinbeutel 20 zugeführt.

Der weitere Abflußschlauch 42 mündet nicht unmittelbar in den Hauptabflußschläuch 18, sondern erst in einem etwas stromabwärts gelegenen Gebiet. Dadurch wird verhindert, daß durch den Hauptabflußschlauch 18 laufender Urin in dem Schlauch 42 hochsteigt und in umgekehrter Richtung durch die Zusatzöffnung 40 austritt. Ferner bewirkt die konstruktive Ausgestaltung, daß bei kräftigem Harnlassen der durch den Abflußschlauch 18 strömende Urin durch Vorbeiströmen an dem Abflußschlauch 42 dort einen Unterdruck ausübt, der am Fuße 38 des Nasenrückens 26 angesammelten Urin über die Zusatzöffnung 40 absaugt.

4

Durch den im vorderen Bereich des Formstückes 10 ausgebildeten Latz 50 wird eine Anlagefläche am Unterleib 70 der Trägerin 74 erzielt, die den Bereich der Scham 68 seitlich und nach oben hin überdeckt. Die Anlagefläche und damit die Sicherheit gegen Verrutschen durch die darübergezogene Kleidung wird somit verbessert.

Eine weitere Stabilisierung in der Lage bewirken die Verlängerungsansätze 44 zu beiden Seiten des Formstückes 10. Diese werden an der Innenseite der Oberschenkel 72 angelegt. Dies kann durch Manschetten oder ein übergezogenes Miederhöschen 73 erfolgen.

Ein solches Miederhöschen 73 bildet dann in Verbindung mit der dargestellten Vorrichtung eine funktionale Einheit, indem auf das Formstück 10, die Verlängerungsansätze 44 und das doppelseitige Klebeband 36 ein Druck ausgeübt wird, der diese Teile an den Körper andrückt. Die dadurch erzielte Haftreibung verhindert auch bei Bewegungen ein Verrutschen der Vorrichtung, da auch bei Bewegungen eines Körperbereichs die anderen in Ruhe bleibenden Körperbereiche die Stabilisierung übernehmen. Dabei wirkt sich vorteilhaft aus, daß bei Verwendung doppelseitigen Klebebandes die Hauptfestigkeit gegen Verrutschen in unmittelbarer Nähe der Harnröhrenöffnung liegt, wodurch auch bei starken Bewegungen kaum eine nennenswerte Verlagerung letzterer Hautbereiche gegenüber der Harnröhren öffnung auftritt.

Zur Erleichterung des Anlegens der erfindgungsgemäßen Vorrichtung dient eine Anlegehilfe, die zunächst bei korrekt angelegtem Formstück 10 kalibriert werden kann. Dies geschieht durch Einhaken der Zapfen 54 in die Löcher 52, Anlegen des Meßstreifens 62 an den Bauch und Einstellen der Markierung 60 auf den Bauchnabel.

Beim Auswechseln der erfindungsgemäßen Vorrichtung kann dann die Anlegehilfe 56 mit ihren Zapfen 54 in die Löcher 52 des neuen Formstücks 10 eingerastet werden, der Meßstreifen 62 mit seiner Markierurig 60 dann an den Bauchnabel angelegt werden und das Formstück 10 an den Unterleib geschwenkt werden. Bei richtiger Handhabung ergibt sich dann eine automatisch richtige Lage der Abflußöffnung 12 gegenüber der Harnröhrenöffnung.

Im Zusammenhang mit der als doppelseitiges Klebeband ausgebildeten Klebeschicht 34 ist auf den Vorteil hinzuweisen, daß die Lage der Klebeschicht 34 durch die doppelseitige Klebewirkung variabel auf dem Formstück 10 ist, wodurch erreicht wird, daß die Abflußöffnung 12 je nach den anatomischen Gegebenheiten der Trägerin 74 jeweils zentral über der Harnröhrenöffnung zu liegen kommt. Diese individuelle Anpassung ermöglicht eine vielseitige Verwendung und eine Optimierung der Lage des Formstückes 10 und der Klebeschicht 34.

## Patentansprüche

1. Urinableitungsvorrichtung für harninkontinente weibliche Personen, bestehend aus einem herzähnlichen, zur Anlage im Schritt und im Bereich der Scham der Trägerin bestimmten Formstück (10), das eine Abflußöffnung (12) sowie eine zur Spreizung der äußeren Schamlippen bestimmte Erhebung aufweist, die als zentral zwischen die äußeren Schamlippen zu liegen kommende Nase (14) ausgebildet ist, welche die Abflußöffnung (12) zum Vaginalbereich hin begrenzt, wobei auf dem Formstück (10) eine die Abflußöffnung (12) mitsamt der Nase (14) umgebende ovale Zone vorgesehen ist, **dadurch gekennzeichnet**, daß auf der genannten Zone (34) eine Klebeschicht (36) zur urindichten Befestigung des Formstückes (10) an dem die äußeren Schamlippen umgebenden Hautbereich der Trägerin angeordnet ist.

2. Vorrichtung nach Anspruch 1, **dadurch gekennzeichnet**, daß die Klebeschicht durch ein doppelseitiges Klebeband (36) gebildet ist.

3. Vorrichtung nach Anspruch 1 und/oder 2, **dadurch gekennzeichnet**, daß innerhalb des von der ovalen Zone (36) umgrenzten Bereichs am Fuß (38) des Nasenrückens (26) eine Zusatzöffnung (40) angeordnet ist, der sich ein weiterer Abflußschlauch (42) anschließt.

4. Vorrichtung nach einem der vorhergehenden Ansprüche 1 - 3, **dadurch gekennzeichnet**, daß sich die Abflußschläuche (18, 42) auf dem Wege zu dem Urinbeutel (20) vereinigen.

5. Vorrichtung nach einem oder mehreren der Ansprüche 1 - 4, **dadurch gekennzeichnet**, daß auf beiden Seiten des Formstückes (10) in Höhe der Nase (14) Verlängerungsansätze (44) angeordnet sind, die zur Anlage an den Innenseiten der Oberschenkel der Trägerin bestimmt sind.

6. Vorrichtung nach Anspruch 5, **dadurch gekennzeichnet**, daß die Verlängerungsansätze (44) einen Zuschnitt besitzen, der sich von einem schmalen Steg (46) zur Befestigung am Formstück in Richtung auf sein freies Ende (48) verbreitert.

7. Vorrichtung nach einem der vorhergehenden Ansprüche 1 - 6, **dadurch gekennzeichnet**, daß die mit der Haut der Trägerin unmittelbar kontakthaltenden Bereiche des Formstückes (10) und der Verlängerungsansätze (44) mit einer Transpiration aufnehmenden Textilie beschichtet sind.

## Claims

1. Device for the discharge of urine for persons of the female sex, suffering from enuresis, consisting of a heartlike form (10), which is intended for putting on in the crotch and within the region of the vulva of the wearer, and which has an outlet (12) also as well as an elevation intended for the spread of the vulva, whereby the elevation is formed like a nose (14) lying central between the vulva and which is bordering the outlet (12) from the region of the vagina whereat on the form is marked out an oval zone surrounding the outlet (12) together with the nose (14), characterized in that on the mentioned zone (34) is arranged a film of adhesive (36) for the urine tight fixing of the form (10) on the skin surrounding the vulva of the wearer.

2. Device according to claim 1, characterized in that the film of adhesive (36) is formed by means of a bilateral adhesive tape.

3. Device according to claim 1 and/ or 2, characterized in that inside the region at the bottom (30) of the bridge of the nose (26) surrounded by the oval zone, is arranged a supplementary opening (40) to which is added a further discharge hose.

4. Device according to one of the preceding claims 1 - 3, characterized in that the discharge hoses (18,42) fuse on the way to the urine bag.

5. Device according to one or several claims 1 - 4, characterized in that on both sides of the form (10) at the hight of the nose (14) are arranged extensions (44) which are intended for wearing at the insides of the thighs.

6. Device according to claim 5, characterized in that the extensions (44) have a cut which get wider from a narrow bridge (46) for fastening at the form towards its free end.

7. Device according to one of the preceding claims 1 - 6, characterized in that the region of the form (10) and the extensions (44) which contact the skin of the wearer are coated with textile absorbing transpiration.

## Revendications

1. Dispositif d'évacuation d'urine pour les personnes de sexe féminin souffrant d'énurésie, constitué d'une pièce moulée (10) en forme de coeur, qui se place entre les jambes, dans la zone de la vulve de la porteuse, et qui présente une ouverture d'évacuation (12), ainsi qu'une élévation destinée à écarter les grandes lèvres, élévation conçue comme un nez central (14), qui se place entre les grandes lèvres et qui limite l'ouverture d'évacuation du côté de la zone vaginale, sur la pièce moulée (10) étant prévue une zone ovale entourant l'ouverture d'évacuation (12) y compris le nez (14), caractérisé en ce que, sur ladite zone ovale (34), est disposée une couche adhésive (36) pour fixer la pièce moulée (10), de manière étanche, sur la peau de la porteuse qui entoure les grandes lèvres.

2. Dispositif suivant revendication 1, caractérisé en ce que la couche adhésive est une bande adhésive des deux côtés (36).

3. Dispositif suivant revendication 1 et/ou 2, caractérisé en ce que, à l'intérieur de la partie délimitée par la zone ovale (36), est disposée, au pied (38) du dos du nez (26), une ouverture complémentaire à laquelle est raccordé un autre tuyau d'évacuation (42).

4. Dispositif suivant une des revendications précédentes 1 à 3, caractérisé en ce que les tuyaux d'évacuation (18, 42) se rejoignent pour déboucher dans la poche à urine (20).

5. Dispositif suivant une ou plusieurs des revendications 1 à 4, caractérisé en ce que, des deux côtés de la pièce moulée (10), sont disposées, au niveau du nez (14), des pièces (44), qui se placent contre les parties intérieures des cuisses de la porteuse.

6. Dispositif suivant revendication 5, caractérisé en ce que les pièces ajoutées (44) présentent une coupe allant en s'élargissant à partir d'un talon étroit (46), pour la fixation à la pièce moulée, vers leurs extrémités libres (48).

7. Dispositif suivant une des revendications précédentes 1 à 6, caractérisé en ce que les zones de la pièce moulée (10) et des pièces ajoutées (44), directement en contact avec la peau de la porteuse, sont recouvertes d'un textile absorbant la transpiration.

Fig. 1

EP 0 304 452 B1

Fig. 2

Fig. 3